# EUROPEAN PATENT APPLICATION

(11) **EP 4 220 502 A1**
(43) Date of publication of application: **02.08.2023**
(21) Application number: 21871868.2
(22) Date of filing: 17.02.2021
(51) Int. Cl.: G06Q 10/00, G01N 35/00

(54) **AUTOMATIC ANALYSIS DEVICE, RECOMMENDED ACTION NOTIFICATION SYSTEM, AND RECOMMENDED ACTION NOTIFICATION METHOD**

(30) Priority: 28.09.2020 JP 2020162044
(71) Applicant: Hitachi High-Tech Corporation, Minato-ku Tokyo 105-6409 (JP)
(72) Inventor: NAKAJIMA, Aika, Tokyo 105-6409 (JP); IIJIMA, Masahiko, Tokyo 105-6409 (JP); IMAI, Kenta, Tokyo 105-6409 (JP); SASAKI, Shunsuke, Tokyo 105-6409 (JP)
(74) Representative: MERH-IP Matias Erny Reichl Hoffmann Patentanwälte PartG mbB
(86) International application number: PCT/JP2021/005819
(87) International publication number: WO 2022/064731

(57) **Abstract**

The present invention provides a system that makes a recommendation, to an operator, of a countermeasure method considered appropriate from a precursory stage of abnormality. This recommended action notification system comprises: a plurality of automatic analyzers 51 that include a first automatic analyzer; and a learning device 52 that is connected to a network 53. The recommended action notification system is for making a recommendation to an operator for an action to be executed on the first automatic analyzer. Said recommended action notification system is provided with: a processing portion 63 that receives specimen analysis result data or maintenance result data from the first automatic analyzer, that inputs related device data including the specimen analysis result data or the maintenance result data to learning models 72, and that, when a probability value that is for recommending execution of a predetermined action and that is outputted by a learning model is equal to or higher than a predetermined threshold value, makes a recommendation to the operator for the predetermined action to be executed on the first automatic analyzer; and an update portion 71 that updates the learning model on the basis of the learning dataset from the plurality of automatic analyzers.

## Description

### TECHNICAL FIELD

The present invention relates to an automatic analyzer, a recommended action notification system, and a recommended action notification method that notify an operator of actions recommended for the automatic analyzer.

### BACKGROUND ART

In recent years, many automatic analyzers have the function of notifying operators of an abnormality detected by the device along with an alarm and presenting a countermeasure predetermined for the alarm. In most cases, however, multiple countermeasures are merely presented. The operator needs to collect device data, identify the cause, and select an appropriate countermeasure from the presented countermeasures.

As described in patent literature 1, the solution to easily troubleshoot the cause of an accuracy management failure uses a chronological graph, for example, that represents information related to the measurement data acquired by a sample analyzer and information related to the operation of the sample analyzer in relation to each other. The purpose is to generate data that enables an operator to identify the states of the sample analyzer at a glance.

### CITATION LIST

### PATENT LITERATURE

PATENT LITERATURE 1: JP-A No. 2019-174424

### SUMMARY OF INVENTION

### TECHNICAL PROBLEM

To determine to take proper countermeasures based on alarms notifying abnormalities from the automatic analyzer, the operator is required to know what device data is needed to identify the cause and have enough experience to estimate the cause from the collected device data and determine countermeasures appropriate for the device in consideration of operational situations. It may be difficult for an inexperienced operator to select appropriate countermeasures.

The method described in patent literature 1 displays information such as accuracy management of standard samples in association with information related to operations of the sample analyzer whereas, conventionally, the former information was merely displayed on screens or paper media. A well-experienced operator can intuitively understand from the displayed content whether the device caused an accuracy management failure. However, it may still be difficult for inexperienced operators to estimate the cause from the displayed information and take appropriate countermeasures.

For example, the automatic analyzer monitors sample measurement results and maintenance results. When the result exceeds a predetermined threshold, the automatic analyzer detects the result as an abnormality and notifies the operator of the abnormality. However, a measurement result may exceed the threshold due to various causes such as abnormalities of the specimen, the reagent, or mechanisms of the device. It is difficult to identify the cause and determine a countermeasure merely by checking the data where the abnormality is detected. Conventionally, when an abnormality is detected, the operator is notified of all the countermeasures concerning the possible causes.

In consideration of the foregoing, the present invention provides a system and a method capable of recommending countermeasures considered appropriate for an operator as early as the stage where an abnormality is predicted.

### SOLUTION TO PROBLEM

A recommended action notification system as one aspect of the present invention includes multiple automatic analyzers including a first automatic analyzer and a learning device networked to the automatic analyzers and recommends an operator an action to be performed on the first automatic analyzer. The recommended action notification system includes a processing portion and an update portion. The processing portion receives sample analysis result data or maintenance result data from the first automatic analyzer, supplies the learning model with related device data including the sample analysis result data or the maintenance result data, and recommends an operator to perform a predetermined action on the first automatic analyzer when a probability value is greater than or equal to a predetermined threshold. The probability value recommends performing the predetermined action output from the learning model. The update portion updates the learning model based on learning datasets from the automatic analyzers.

### ADVANTAGEOUS EFFECTS OF INVENTION

There is provided a system and a method capable of recommending countermeasures considered appropriate for an operator as early as the stage where an abnormality is predicted.

other objects and novel features of the invention will be apparent from the following description of the specification taken in connection with the accompanying drawings.

### BRIEF DESCRIPTION OF DRAWINGS

Fig. 1A is a diagram illustrating a whole view of the recommended action notification system;
Fig. 1B is a functional block diagram of the recommended action notification system;
Fig. 2 is a schematic diagram of an automatic analyzer;
Fig. 3 is a flowchart to update a learning model;
Fig. 4 illustrates an action evaluation input screen;
Fig. 5 illustrates related device data;
Fig. 6 illustrates a learning model using a neural network;
Fig. 7 illustrates an operation table;
Fig. 8 is a flowchart to output recommended actions through the use of a learning model;
Fig. 9 illustrates a recommended action display screen;
Fig. 10 illustrates a learning model for action of "light source lamp replacement;"
Fig. 11 illustrates chronological absorbance data as photometer check data;
Fig. 12 illustrates reaction monitor data;
Fig. 13 illustrates a recommended action display screen;
Fig. 14 illustrates a learning model for action of "sample probe outer wall cleaning;"
Fig. 15 illustrates a measurement result of failing to clean the sample probe outer wall;
Fig. 16A illustrates reaction monitor data (ALB reaction process) in a case of failing to clean the sample probe outer wall;
Fig. 16B illustrates reaction monitor data (CRE reaction process) in a case of failing to clean the sample probe outer wall; and
Fig. 17 illustrates a recommended action display screen.

### DESCRIPTION OF EMBODIMENT

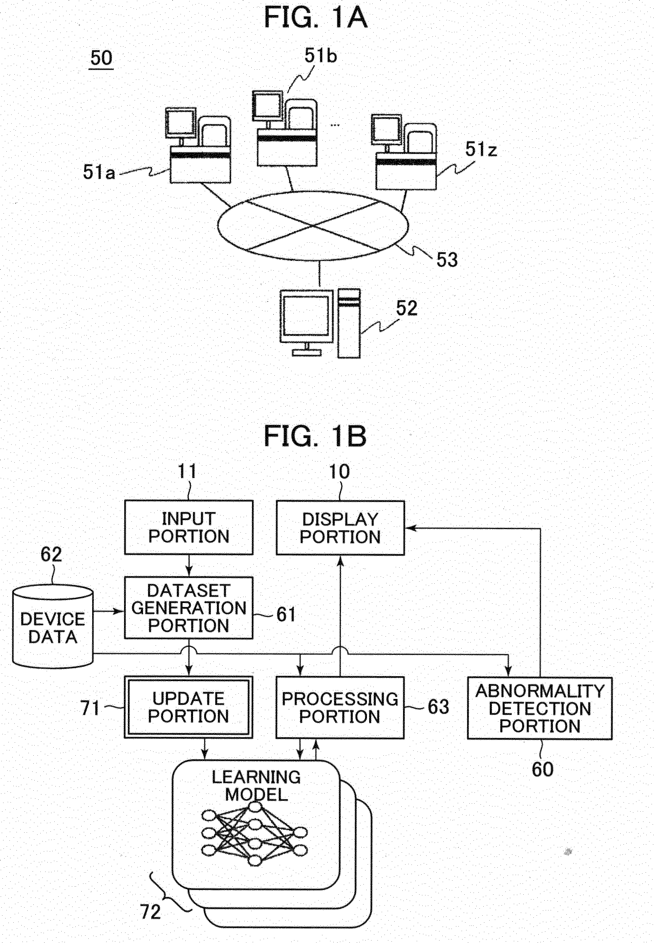

Fig. 1A is a diagram illustrating a whole view of a recommended action notification system 50 according to the present embodiment. The recommended action notification system 50 includes multiple automatic analyzers 51a through 51z and a learning device 52 connected via a network 53. The automatic analyzer 51 is installed in clinical laboratories or a clinical center, for example. There is no limit to the number of automatic analyzers 51 connected to the learning device 52. The learning device 52 as hardware is an information processor including a storage device such as HDD (Hard disk drive) or SSD (Solid State Drive). As will be described later, Fig. 1B shows a functional block diagram illustrating the functions provided by the automatic analyzer 51 or the learning device 52. To implement each function, the program codes of the software are stored in the main memory. Then, the processor executes the stored program codes. The present embodiment may use the term "portion" to express a function the information processor implements based on the program. When the learning device 52 processes large-scale data, the data itself need not be saved in the storage device of the learning device 52. For example, the data may be saved in a cloud object storage and a data path to access the target data may be stored. The learning device 52 is not limited to physical configurations of the hardware and may be embodied as one server or distributed processing servers.

Fig. 1B is a functional block diagram illustrating an overview of the recommended action notification system 50. As will be described in detail later, the automatic analyzer 51 allows a dataset generation portion 61 to generate a learning dataset from not only action effect information entered by a device operator from an input portion 11, but also device data stored in a database 62. The learning dataset includes the action effect information and action-related device data (related device data). As will be described later, an action to generate the learning dataset is assumed to be triggered by an abnormality detection portion 60 that detects an abnormality from the sample measurement result or maintenance result and notifies the operator of the abnormality (by displaying it on a display portion 10, for example).

The learning dataset generated by the dataset generation portion 61 is transmitted to the learning device 52 via the network 53. In the learning device 52, an update portion 71 generates a training signal based on the learning dataset and updates a learning model 72. The updated learning model 72 is delivered to the automatic analyzer 51 via the network 53. Each time a sample measurement result or a maintenance result is output, the automatic analyzer 51 allows a processing portion 63 to input the related device data stored in the database 62 to the learning model 72 and output a recommended action. The display portion 10 of the automatic analyzer 51 displays the recommended action to be notified to the operator.

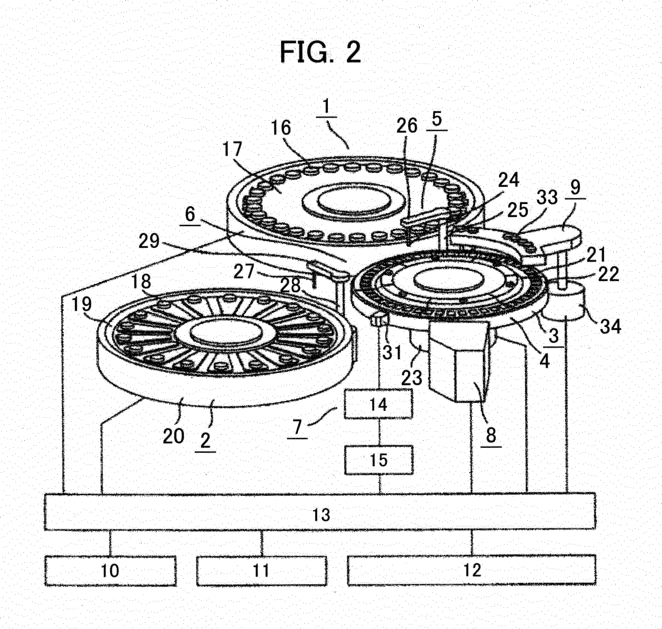

Fig. 2 illustrates an overview of the automatic analyzer 51. The automatic analyzer 51 mainly includes a sample disk 1, a reagent disk 2, a reaction disk 3, a reactor 4, a sampling mechanism 5, a pipetting mechanism 6, a stirring mechanism 7, a photometric mechanism 8, a cleaning mechanism 9, a display portion 10, an input portion 11, a storage portion 12, and a control portion 13. Multiple sample containers 16 containing collected samples are stationarily placed on the circumference of a circular disk 17 of the sample disk 1. The circular disk 17 rotates to be positionable in the circumferential direction based on a drive mechanism composed of a motor and a rotating shaft (not shown). Multiple reagent bottles 18 contain reagents to mix and react with samples and are stationarily placed on the circumference of a circular disk 19 of the reagent disk 2 that is surrounded by a temperature-controlled cooler 20. The circular disk 19 rotates to be positionable in the circumferential direction based on a drive mechanism composed of a motor and a rotating shaft (not shown). Multiple reaction container holders 22 are attached to the reaction disk 3 and hold a reaction container 21 that is used to contain samples and reagents. A drive mechanism 23 causes the reaction disk 3 to repeatedly start and stop rotating in the circumferential direction at a specified cycle to intermittently transfer the reaction container 21. The reactor 4 is installed along the trajectory along which the reaction container 21 moves. When the reaction container 21 contains reaction liquid, a mixture of sample and reagent, the reactor 4 functions as a constant temperature reservoir that keeps the reaction liquid at a constant temperature through the use of temperature-controlled constant temperature water, for example, to promote the chemical reaction between the sample and the reagent. The reaction container 21 moves inside the reactor 4. The sampling mechanism 5 includes a probe 24, an arm 26 attached to a bearing shaft 25, and a drive mechanism that can reciprocate between the sample disk 1 and the reaction disk 3 around the bearing shaft 25. The sampling mechanism 5 supplies the sample in the sample container 16 to the reaction container 21 according to a predetermined sequence while the sample disk 1 rotates to transport the sample container 16 to a predetermined position. Similarly, the pipetting mechanism 6 includes a probe 27, an arm 29 attached to a bearing shaft 28, and a drive mechanism that can reciprocate between the reagent disk 2 and the reaction disk 3 around the bearing shaft 28. The pipetting mechanism 6 supplies the reagent in the reagent bottle 18 to the reaction container 21 according to a predetermined sequence while the reagent disk 2 rotates to transport the reagent bottle 18 to a predetermined position. The sample containers 16 and the reagent bottles 18 contain different types of samples and reagents, respectively. The required amount thereof is supplied to the reaction container 21. The stirring mechanism 7 stirs and mixes the sample and reagent in the reaction container 21 transferred to that position (stirring position). The stirring mechanism 7 uses the non-contact stirring technique and includes a securing portion31, a piezo element driver 14, and a stirring mechanism controller 15. The securing portion 31 secures a piezoelectric element (not shown) to the position capable of irradiating a sound wave from the side of the reaction container 21 at the stirring position. The piezo element driver 14 drives the piezoelectric element.

The control portion 13 sends control signals to the sample disk 1, the reagent disk 2, the reaction disk 3, the reactor 4, the sampling mechanism 5, the pipetting mechanism 6, the stirring mechanism 7, the photometric mechanism 8, and the cleaning mechanism 9 to control their operations. The control portion 13 also performs the functions of the recommended action notification system according to the present embodiment. Specifically, the control portion 13 performs the functions of the abnormality detection portion 60, the dataset generation portion 61, and the processing portion 63 illustrated in Fig. 1B. The display portion 10 provides various displays concerning analysis items and results on the screen. The input portion 11 inputs various information such as analysis items. The storage portion 12 stores various information such as analysis items and predetermined sequences (programs) to control the mechanisms. The storage portion 12 also stores the updated learning model 72 delivered from the database 62 and the learning device 52 illustrated in Fig. 1B.

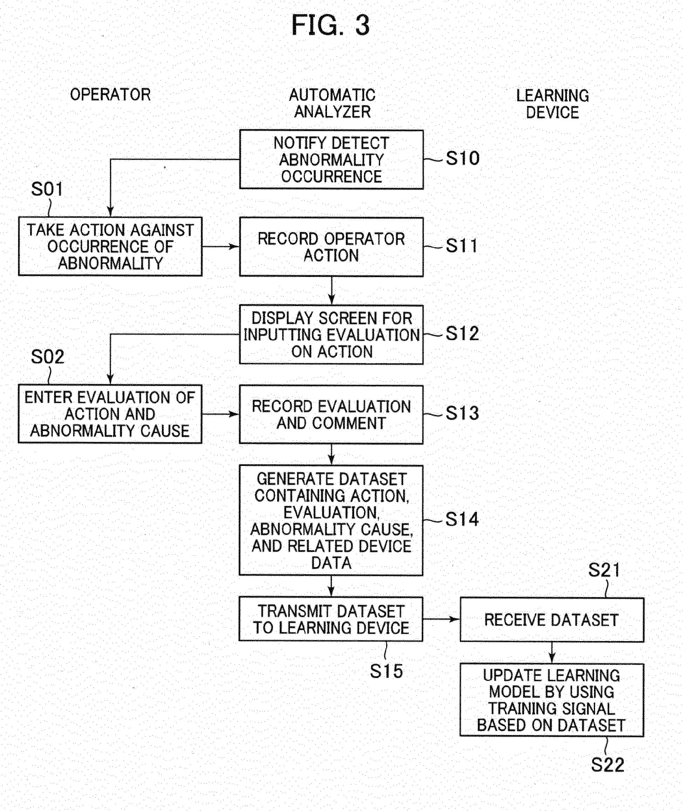

Fig. 3 illustrates a flowchart to update the learning model 72. The learning model 72 uses different inputs and outputs depending on the actions to be learned. The recommended action notification system 50 individually generates a learning model according to the action. The system selects actions to be learned. Specifically, actions to be learned are assumed to be the operator's operations such as carrying out the maintenance, replacing the reagent, and remeasuring the sample dilution to be performed when a device abnormality occurs.

The learning model is assumed to be initially learned based on training signals such as device abnormality data previously collected by the recommended action notification system 50, the corresponding recommended action, and the cause of the abnormality.

The abnormality detection portion 60 of the automatic analyzer 51 notifies the operator of an alarm when detecting an abnormality from the result of the sample test performed by the device or the result of maintenance (S10). The operator takes action against the abnormality (S01). The control portion 13 of the automatic analyzer records the operator's action as a device operation log (S11). It may be determined from the operation log that the operator took the action to be learned. Then, the dataset generation portion 61 of the automatic analyzer causes the display portion 10 to display an input screen for inputting the effectiveness evaluation on the operator's action after the action is taken (S12). The present flowchart takes effect when the abnormality detection portion 60 detects an occurrence of some abnormality in the device and triggers the operator to take an action. Regular maintenance actions are not collected as learning datasets. This is because the learning can be optimized by collecting action as a learning dataset only when the action provides an obvious effect or not.

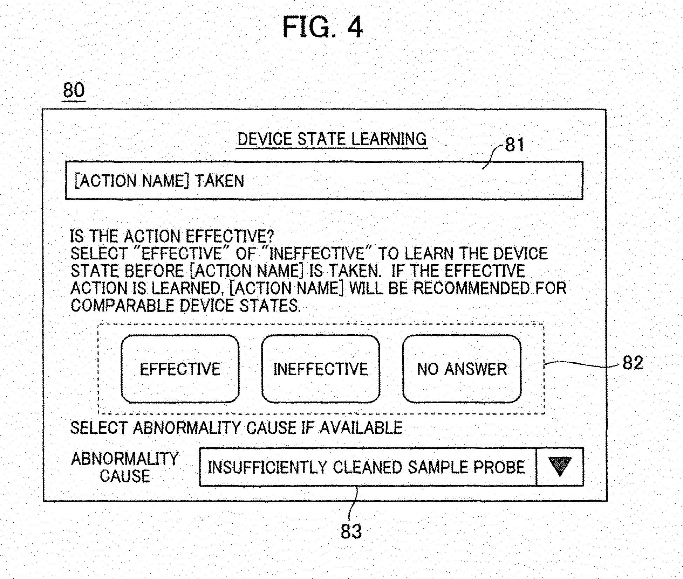

Fig. 4 illustrates an action evaluation input screen 80. An action content display portion 81 displays the action taken by the operator. [Action name] in the drawing displays the name of the action taken by the operator. The operator selects one of effect buttons 82 to enter the effectiveness evaluation of the action (S02). An abnormality cause selection portion 83 provides the abnormality cause identified by the operator's action (S02). The list box is used for input to reduce the operator's input effort and inhibit variations in the expressions. The storage portion 12 of the automatic analyzer 51 records action evaluations and abnormality causes entered by the operator (S13).

For each action, the dataset generation portion 61 generates a learning dataset that contains the operator-entered evaluation, the abnormality cause, and related device data (S14). The generated learning dataset is transmitted to the learning device 52 (S15).

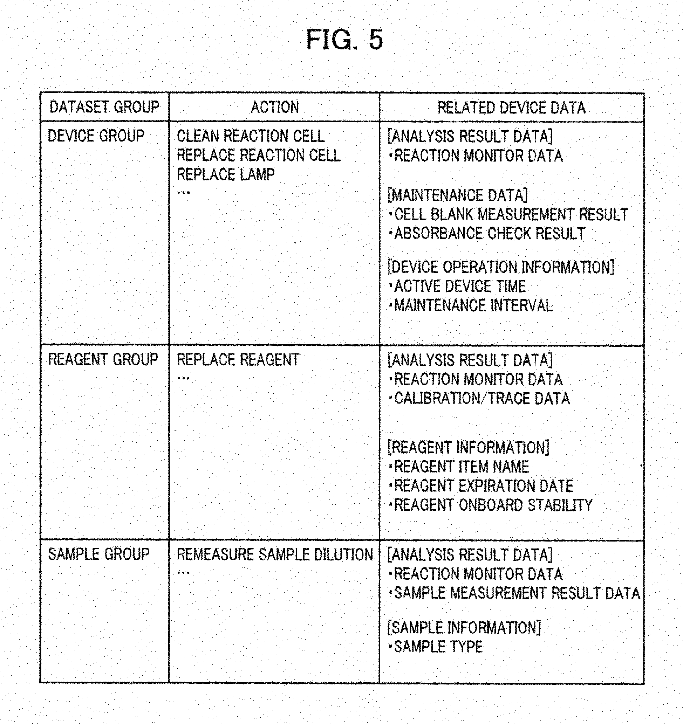

To update the learning model, the related device data is collected for a predetermined period based on the date and time when the operator's action was taken. Fig. 5 illustrates the related device data. The related device data is not limited to hardware but widely includes information about the mechanisms, hardware, reagents, and samples related to device operations, and analysis result data, for example. The related device data is settled for each action because device data symptomizing an abnormality depends on the abnormality (abnormality cause) for which the operator's action is effective. Fig. 5 illustrates dataset groups that categorize the types of related device data settled for the corresponding actions. The dataset group categorizes actions according to the action contents. The three dataset groups "device," "reagent," and "sample" correspond to actions related to devices, reagents, and samples, respectively, and are highly common to the types of related device data to be collected.

Favorably, the learning dataset contains time information about actions. The time information includes the date and time to take the action and the date and time of an occurrence of the abnormality (error) detected by the abnormality detection portion 60 while the action taken against that abnormality was proven to be effective.

The update portion 71 of the learning device 52 receives the learning dataset (S21) and updates the learning model 72 by using the training signal based on the received learning dataset (S22). The updated learning model is distributed to the automatic analyzer 51 at a predetermined timing.

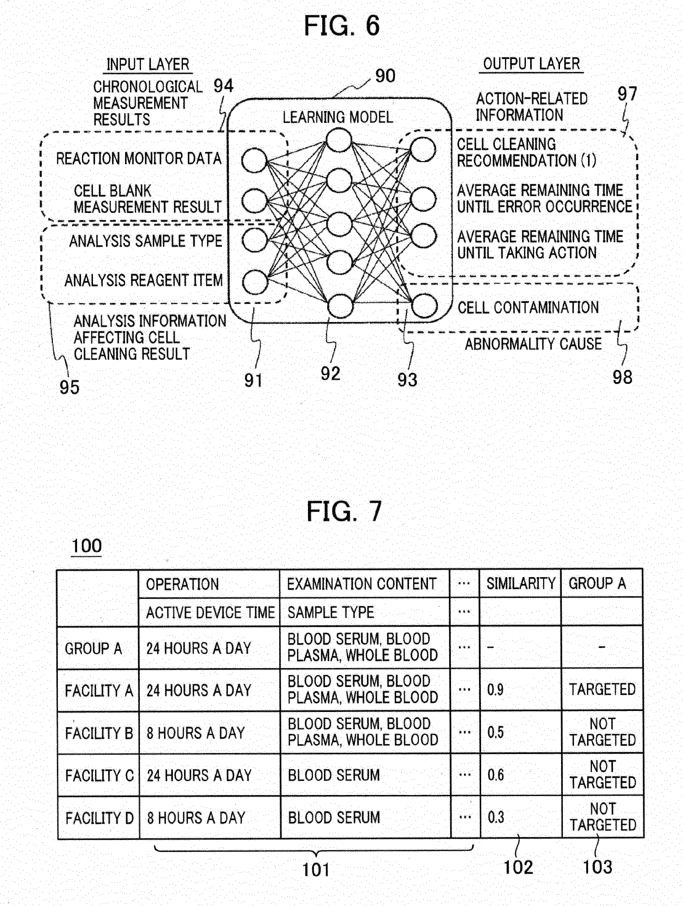

The learning model 72 can use any learning device such as a neural network, a regression tree, or a Bayes classifier. Fig. 6 illustrates a learning model using the neural network. In the learning model 90, information input to an input layer 91 is propagated to an intermediate layer 92 and then to an output layer 93 in order. The output layer 93 outputs inference results based on the information input to the input layer 91. The input layer 91, the intermediate layer 92, and the output layer 93 respectively include multiple input units, intermediate units, and output units indicated by circles. In the drawing, the intermediate layer 92 is shown as a representative while the neural network usually uses multiple intermediate layers. Information input to each input unit in the input layer 91 is weighted by a coupling coefficient between the input unit and the intermediate unit and is input to each intermediate unit. Values of the intermediate units in the intermediate layer 92 are calculated by adding values from the input units. Output from each intermediate unit in the intermediate layer 92 is weighted by a coupling coefficient between the intermediate unit and the output unit and is input to each output unit. Values of the output units in the output layer 93 are calculated by adding values from the intermediate units. As above, processing in the intermediate layer 92 is comparable to the non-linear transformation of the input data values input to the input layer 91 and the output of the values as output data in the output layer 93.

Fig. 6 illustrates the learning model 90 concerning an action of "reaction cell cleaning" along with input/output data examples.

The input layer 91 is supplied with related device data or its processed data which is also hereinafter referred to as the related device data without distinction. The output layer 93 outputs a calculation result corresponding to the input. The related device data input to the input layer 91 includes a chronological measurement result 94 such as an analysis result and maintenance data defined for each learning model; and analysis information 95 such as reagent information and sample information that affect actions. The output data from the output layer 93 is an inference result concerning the action of "reaction cell cleaning" and includes action-related information 97 and an abnormality cause 98. The action-related information 97 includes the determination of whether to recommend an action (such as "reaction cell cleaning"), the average remaining time until error occurrence, and the average remaining time until action implementation. The average remaining time until error occurrence signifies the average remaining time required for the abnormality detection portion 60 to detect an abnormality (error) corresponding to the action. The average remaining time until action implementation signifies the average remaining time required for the operator to take action. The average remaining time until error occurrence provides a guide to know the approximate time when the action will be needed in the future even if the action is not recommended. The remaining time until action implementation further reflects operations at each facility and operator's experience values and provides a guide to know the approximate time when the action will be generally taken in the future.

Output values from the output units such as "recommendation of reaction cell cleaning (action)" and "abnormality cause (cell contamination)" provide probability values. It is determined that the action is recommended or the abnormality cause is notified depending on whether the output probability value is greater than or equal to or is smaller than a predetermined threshold. For example, the reaction cell cleaning is recommended as the output unit of "recommendation of reaction cell cleaning" approximates 1, or the same is not recommended as the output unit thereof approximates 0. Similarly, the abnormality cause is likely to be true as the output unit of "abnormality cause (cell contamination)" approximates 1, or the same is unlikely to be true as the output unit thereof approximates 0. The output unit of "average remaining time until error occurrence" and the output unit of "average remaining time until action implementation" directly output values of the remaining time.

In a learning model update process (S22), the update portion 71 generates a training signal corresponding to the input/output data for the learning model 90 based on the learning dataset. An input value for the training signal is the related device data. The training signal generates the following output values in this example. The output value for "recommendation of reaction cell cleaning" is set to 1 if the effectiveness evaluation of the action is effective. The output value for "recommendation of reaction cell cleaning" is set to 0 if the same is ineffective. The output value for "abnormality cause (cell contamination)" is set to 1 if the abnormality cause is "cell contamination." Otherwise, the output value for "abnormality cause (cell contamination)" is set to 0. Concerning the time information, an output value for the training signal is generated by calculating the time elapsed from the predetermined reference time such as the error occurrence date and time or the action implementation date and time. The predetermined reference time may be defined as the implementation date and time of the previous action, for example.

The update portion 71 adjusts coupling coefficient values among the units so that the input value (related device data) for the created training signal is input to the input layer 91 and a value output from the output layer 93 equals the output value for the training signal.

Many learning datasets need to be acquired to improve the accuracy of the learning model 72. To improve the learning effect, however, it is favorable to build a learning model through the use of learning datasets from the automatic analyzers 51 that operate similarly. The automatic analyzers 51 differ from each other in operations and test contents. The automatic analyzers whose operations and test contents are similar may indicate occurrences of common abnormalities and may provide more reliable countermeasures against abnormalities. Different operations of the devices are related to occurrences of different abnormalities. For example, frequent lamp replacement is required for the automatic analyzer in a facility where the devices operate for a long time. Alternatively, probes need to be washed frequently for the automatic analyzer in a facility that analyzes plasma as well as serum, compared to a facility that analyzes only serum.

The accuracy of the learning model can be further improved by subdividing the learning model 72 into each group of facilities in similar operational situations. Fig. 7 illustrates an operation table 100. The learning device 52 collects information about the operational situations of the automatic analyzer in each facility and manages the information based on the operation table 100. Typical operations are defined as a group (group A in this example). It is determined whether the facilities are targeted for the group, based on the similarity between the operational situation defined for the group and the operational situation of each facility. An operational situation information column 101 includes operational situation information defined for the group and operational situation information for each facility. A similarity column 102 includes a correlation value between the operational situation defined for the group and the operational situation of each facility. A target determination column (group A) 103 indicates whether the facility is targeted for the group (group A) that is determined based on the correlation value. In this example, facility A is targeted for group A, and the other facilities are not targeted. It is possible to build a more reliable learning model through the learning by collecting data from the automatic analyzers whose operation forms are highly similar. The condition of the group may be defined as not only the similarity among all items of the operational situation but also the similarity among one or more items of the operational situation depending on abnormality causes.

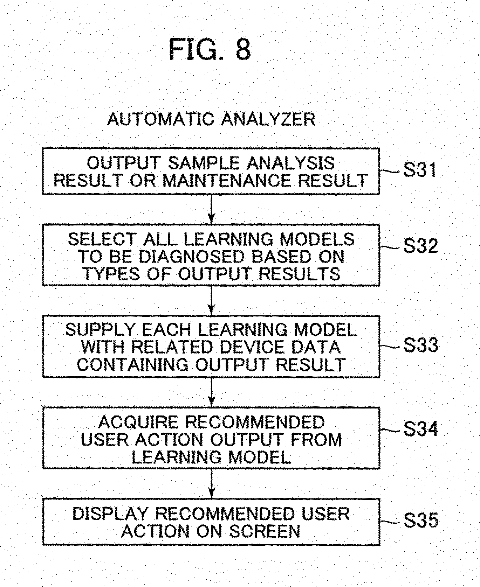

Fig. 8 illustrates a flowchart in which the automatic analyzer 51 uses the learning model 72 to output recommended actions. It is favorable to perform a real-time diagnosis by using the learning model each time the automatic analyzer 51 outputs a sample analysis result or a maintenance result (S31), for example. This makes it possible to early detect a device abnormality and notify the operator of the abnormality. The processing portion 63 selects a learning model to be diagnosed (S32). It is favorable to extract the learning model whose related device data equals the output result of the automatic analyzer 51. Multiple learning models may be extracted. The extracted learning model is supplied with related device data containing the output result corresponding to the learning model (S33). An inference result such as a recommended user action output from the learning model is acquired (S34) and is displayed on the screen (S35).

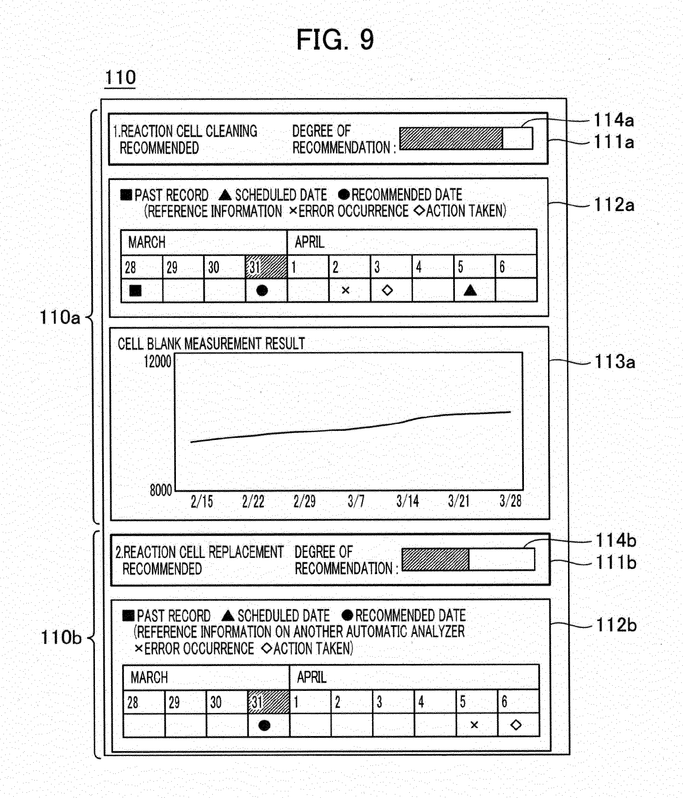

Fig. 9 illustrates a recommended action display screen on the display portion 10. The recommended action display screen 110 includes a recommended action display portion 111, a recommended date display portion 112, and a reference information display portion 113. As above, whether to recommend the action, namely, the output value from the learning model, is determined based on whether the probability value output from the learning model is greater than or equal to, or smaller than a predetermined threshold. One or more actions may be recommended. When multiple actions are recommended, the degree of recommendation can be determined based on the probability value. The screen displays a highly prioritized recommended action, namely, a recommended action indicating a large difference between the threshold value and the probability value. This example uses a bar 114 to display the degree of recommendation for each recommended action and displays the contents of recommended actions in descending order of priorities. According to the example, the preceding recommended action display screen 110a displays the more highly prioritized action of "reaction cell cleaning." The succeeding recommended action display screen 110b displays the action of "reaction cell replacement."

The recommended action display portion 111 displays the name of the recommended action and its degree of recommendation. A larger hatched portion of the bar 114 indicates a higher degree of recommendation.

The recommended date display portion 112 displays the history of actions in the automatic analyzer and the date and time of a scheduled action, if any, in addition to the output values from the learning model. The shaded date in the calendar represents today, indicating that the action of "reaction cell cleaning" is recommended through the use of a black circle. The display also includes reference information such as the date and time to indicate the error occurrence date or to take the action in general automatic analyzers, based on the estimation of the average remaining time output from the learning model. It is possible to determine whether the operator performs the recommended action, based on general or average timings.

The reference information display portion 113 can display device data highly important to determine whether to require a predetermined recommended action, namely, the history of reaction cell blank measurement data for the reaction cell cleaning, for example. It is possible to confirm whether the recommended action is appropriate in consideration of the operator's experience, without the need for the operator to newly collect the device data.

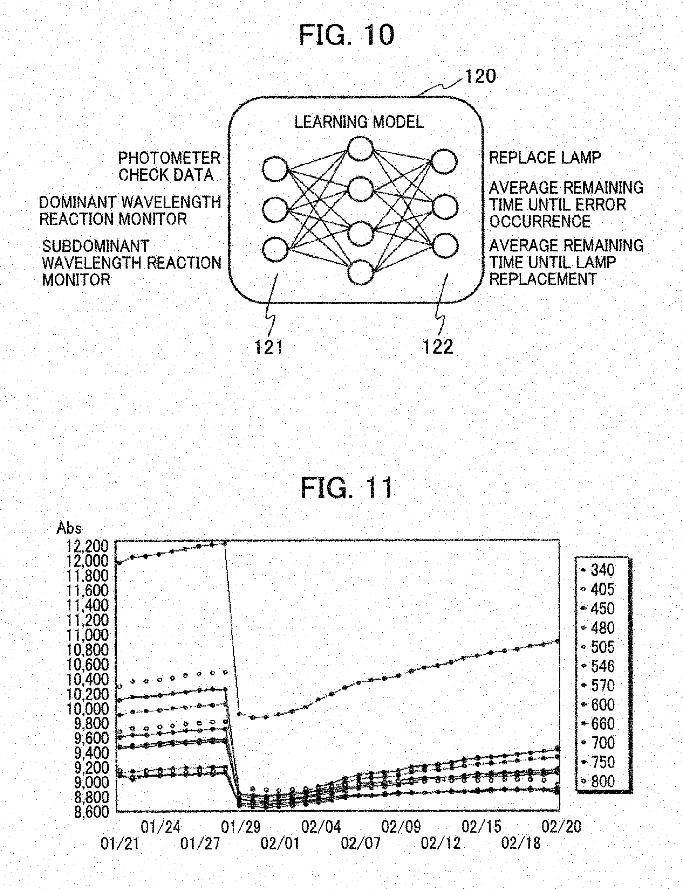

Figs. 10 to 12 illustrate an example of learning the action of "light source lamp replacement." Generally, the light source lamp is replaced at regular intervals or after a predetermined period of usage, whichever is reached first, or when the photometer check value exceeds a threshold. The lamp does not necessarily burn out even after the photometer check value exceeds the replacement threshold if defined. It is recommended to replace the lamp periodically or after the lamp is used for a predetermined period or longer. Even though the regular replacement is recommended, however, appropriate periods depend on the active device time in each facility. For example, the lamp needs to be replaced more frequently in a facility using the device ten hours a day than in a facility using the device five hours a day. The following description assumes that the learning dataset used for learning uses data collected from the automatic analyzers in facilities whose operating hours are approximate to each other.

The example learning model in Fig. 10 collects a learning dataset including the related device data and recommends effective timing to replace the light source lamp. The related device data includes the chronological measurement data concerning the light source lamp; and the action-related time information such as the timing of an abnormality to occur in the measurement data due to the light source lamp and the timing for the operator to replace the light source lamp. The input layer 121 of the learning model 120 is supplied with the related device data such as the chronological photometer check data of the light source lamp; and the chronological reaction monitor data for each of the dominant wavelengths and subdominant wavelengths. The output layer 122 outputs the determination of whether to recommend replacing the light source lamp and the reference information such as the average remaining time until an abnormality occurrence in the reaction monitor data and the average remaining time until the light source lamp replacement concerning the automatic analyzers in facilities indicating similar device operations.

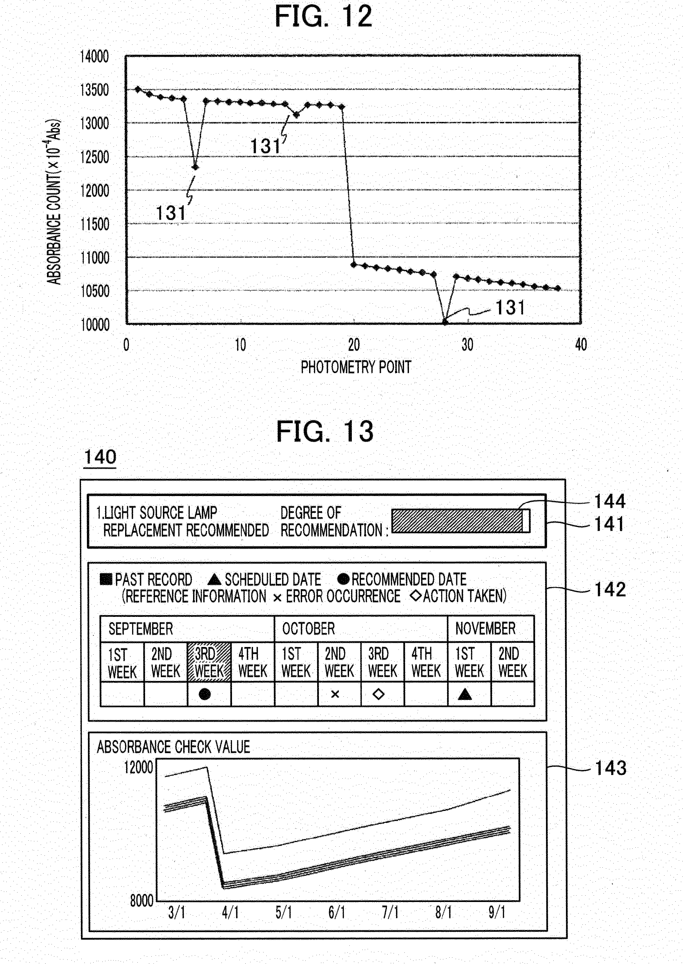

Fig. 11 illustrates chronological absorbance data as an example of the photometer check data used for learning or inference. The light source lamp tends to decrease the light intensity and gradually increase the absorbance with the lapse of time after the light source lamp is replaced by a new one. Fig. 12 illustrates reaction monitor data used for the learning or inference. A decrease in the life of the light source lamp makes the amount of light unstable, generates a spike noise 131, a sudden change in the absorbance during the reaction process, and causes a linearity abnormality. The spike noise 131 tends to increase with the lapse of time. The learning model 120 infers whether to recommend replacing the light source lamp based on such feature quantities from the chronological data.

The learning of the learning model 120 and the inference using the learning model 120 are equal to those of the action of "reaction cell cleaning." Duplicate explanations will be omitted. Fig. 13 illustrates a recommended action display screen 140. The recommended action display screen 140 includes a recommended action display portion 141, a recommended date display portion 142, and a reference information display portion 143. The display portions are equal to those illustrated in Fig. 9. Duplicate explanations will be omitted. The operator can replace the light source lamp at effective timings based on: the determination of whether to recommend replacing the light source lamp based on the chronological data in the related device data; and the average error occurrence timing and the operator's replacement timing on the similarly operating automatic analyzers.

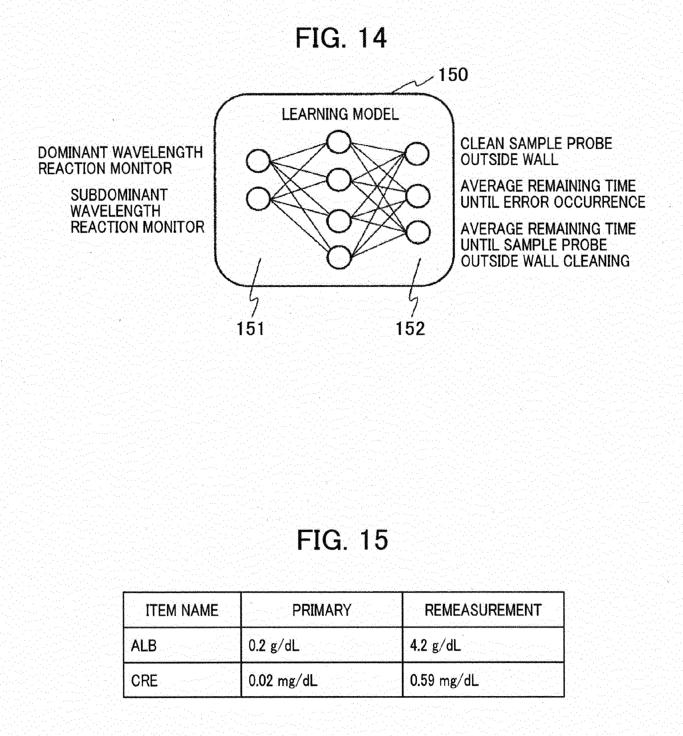

Figs. 14 through 17 illustrate an example of learning the action of "sample probe outside wall cleaning." The outside wall of the sample probe needs to be cleaned daily after the measurement is complete. Failure to clean the outside wall may decrease the sample dispensing accuracy. The decreased sample dispensing accuracy affects sample measurement data. However, the measurement data alone cannot determine whether an abnormality is caused by the sample originally indicating an abnormal value or a decrease in the sample dispensing accuracy. Here, the user is asked to input whether cleaning of the outside wall of the sample probe was effective for the abnormal measurement data. If the cleaning was effective, it is possible to estimate that the abnormality was caused by the decrease in the sample dispensing accuracy. Based on the thus collected learning dataset, a learning model is generated from the measured data to recommend cleaning the outside wall of the sample probe. In this case, the degree of contamination of the sample probe depends on the type of sample used (serum, plasma, or whole blood) or the operation time (such as 24 hours a day). The following description assumes that the learning dataset used for learning contains data collected from the automatic analyzers in facilities subject to similar operational situations that affect the degree of contamination of the sample probes.

The example learning model in Fig. 14 collects a learning dataset including the related device data and recommends effective timing to clean the outside wall of the sample probe. The related device data includes the chronological reaction monitor data for dominant and subdominant wavelengths and the time information about the action such as the timing of the occurrence of an abnormality in the reaction monitor data due to contamination on the sample probe outside wall and timing for the operator to clean the sample probe outside wall. The input layer 151 of the learning model 150 is supplied with the related device data, namely, the chronological reaction monitor data for each of the dominant and subdominant wavelengths. The output layer 152 outputs the determination of whether to recommend cleaning the sample probe outside wall and the reference information such as the average remaining time until the appearance of the sign of an abnormality in the reaction monitor data and the average remaining time until cleaning the sample probe outside wall concerning the automatic analyzers in facilities indicating similar device operations.

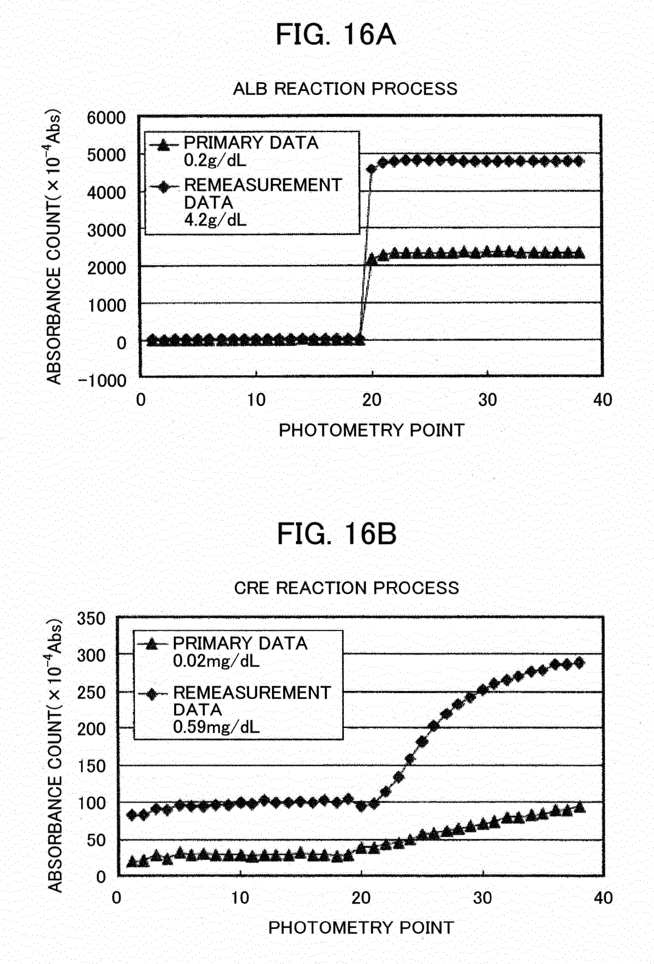

Fig. 15 illustrates a measurement result from the failure to clean the outside wall of the sample probe. Figs. 16A and 16B illustrate reaction monitor data due to the failure to clean the outside wall of the sample probe. Fig. 16A illustrates the monitor data for an ALB reaction process. Fig. 16B illustrates the monitor data for a CRE reaction process. A decrease in the sample dispensing accuracy causes different measurement results from the initial data and remeasured data of the same sample in terms of multiple items. The learning model 150 identifies such feature quantities from chronological data for the reaction process and thereby infers the determination of whether to recommend cleaning the sample probe outside wall.

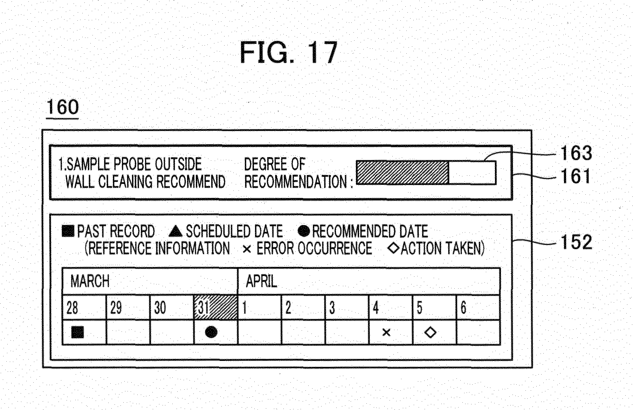

The learning of the learning model 150 and the inference using the learning model 150 are equal to those of the action of "reaction cell cleaning." Duplicate explanations will be omitted. Fig. 17 illustrates a recommended action display screen 160. The recommended action display screen 160 includes a recommended action display portion 161 and a recommended date display portion 162. The display portions are equal to those illustrated in Fig. 9. Duplicate explanations will be omitted. The operator can clean the sample probe outside wall at effective timings based on: the determination of whether to recommend cleaning the sample probe outside wall based on the chronological data in the related device data; and the average error occurrence timing and the operator's replacement timing on the similarly operating automatic analyzers. The recommended action display screen 160 may include a reference information display portion to display chronological data as illustrated in Fig. 16A.

There has been described present invention based on the preferred embodiment and modifications. However, the invention is not limited to the above-described embodiment and modifications but may be otherwise variously modified within the spirit and scope of the invention. According to the above-described example, the recommended action notification system includes multiple automatic analyzers and learning devices. Alternatively, the recommended action notification system may include a single automatic analyzer. However, the use of learning datasets from multiple automatic analyzers for learning makes it possible to fast collect information from many automatic analyzers and promote learning effects. Moreover, the collection of training data from other automatic analyzers makes it possible to acquire inference results from an average automatic analyzer as described above and use the results to determine whether to take the recommended action. According to the above-described example, the automatic analyzer performs inference by using learning models. Alternatively, the processing portion 63 may be provided for the learning device or other information processors to perform the inference.

The recommended action notification system may be configured to give notifications to the operator through the use of a mobile terminal such as a smartphone. The operator can be notified of a recommended action, if any, without restrictions on the operator's locations. In addition to input to the screen, other input methods such as voice input may be used to reduce the operator's effort to input action effects.

### REFERENCE SIGNS LIST

- 1:: sample disk
- 2:: reagent disk
- 3:: reaction disk
- 4:: reactor
- 5:: sampling mechanism
- 6:: pipetting mechanism
- 7:: stirring mechanism
- 8:: photometric mechanism
- 9:: cleaning mechanism
- 10:: display portion
- 11:: input portion
- 12:: storage portion
- 13:: control portion
- 14:: piezo element driver
- 15:: stirring mechanism controller
- 16:: sample container
- 17, 19:: circular disk
- 18:: reagent bottle
- 20:: cooler
- 21:: reaction container
- 22:: reaction container holder
- 23:: drive mechanism
- 24, 27:: probe
- 25, 28:: bearing shaft
- 26, 29:: arm
- 31:: securing portion
- 51:: automatic analyzer
- 52:: learning device
- 53:: network
- 60:: abnormality detection portion
- 61:: dataset generation portion
- 62:: database
- 63:: processing portion
- 71:: update portion
- 72:: learning model
- 80:: action evaluation input screen
- 81:: action content display portion
- 82:: effect button
- 83:: abnormality cause selection portion
- 90, 120, 150:: learning model
- 91, 121, 151:: input layer
- 92:: intermediate layer
- 93, 122, 152:: output layer
- 94:: chronological measurement result
- 95:: analysis information
- 97:: action-related information
- 98:: abnormality cause
- 100:: operation table
- 101:: operational situation information column
- 102:: similarity column
- 103:: target determination column
- 110, 140, 160:: recommended action display screen
- 111,: 141, 161: recommended action display portion
- 112,: 142, 162: recommended date display portion
- 113, 143:: reference information display portion
- 114, 144, 163:: bar
- 131:: spike noise

## Claims

1. A recommended action notification system that includes a plurality of automatic analyzers including a first automatic analyzer and a learning device networked to the automatic analyzers, and recommends an operator an action to be performed on the first automatic analyzer, the recommended action notification system comprising:
a processing portion that receives one of sample analysis result data and maintenance result data from the first automatic analyzer, supplies a learning model with related device data including one of the sample analysis result data and the maintenance result data, and recommends an operator to perform a predetermined action on the first automatic analyzer when a probability value is greater than or equal to a predetermined threshold, wherein the probability value recommends performing the predetermined action output from the learning model; and
an update portion that updates the learning model based on learning datasets from the automatic analyzers.

2. The recommended action notification system according to claim 1,
wherein the first automatic analyzer includes the processing portion and the learning device includes the update portion; and
wherein the learning model updated by the update portion is delivered to the first automatic analyzer.

3. The recommended action notification system according to claim 1,
wherein the learning model includes an input layer supplied with related device data configured according to the predetermined action and an output layer to output an inference result related to the predetermined action in response to input of the related device data to the input layer; and
wherein the inference result includes a probability value to recommend performing the predetermined action and a probability value to notify occurrence of a predetermined abnormality for which the predetermined action is effective.

4. The recommended action notification system according to claim 3,
wherein, when an operator is notified of the detection of an abnormality occurrence from the automatic analyzer and performs the predetermined action, a dataset generation portion of the automatic analyzer displays an action evaluation input screen on a display portion of the automatic analyzer, collects related device data corresponding to the predetermined action during a predetermined period based on the date and time to perform the predetermined action, and generates a learning dataset including the collected related device data and effectiveness evaluation and an abnormality cause of the predetermined action input from the action evaluation input screen.

5. The recommended action notification system according to claim 4,
wherein the inference result includes an average remaining time until the predetermined abnormality occurs on the automatic analyzers and an average remaining time until the predetermined action is performed on the automatic analyzers; and
wherein a dataset generation portion of the automatic analyzer allows the learning dataset to further include a time elapsed from a predetermined reference time until the predetermined abnormality occurs on the automatic analyzer and a time elapsed from a predetermined reference time until the predetermined action is performed on the automatic analyzer.

6. The recommended action notification system according to claim 3,
wherein the processing portion extracts the learning models whose input layer is supplied with one of the sample analysis result data and the maintenance result data defined as related device data; and
wherein the processing portion supplies the extracted learning models with related device data including one of the sample analysis result data and the maintenance result data and, when a probability value recommends performing a predetermined action and there is a plurality of learning models that output a probability value greater than or equal to a predetermined threshold, recommends an operator a plurality of predetermined actions corresponding to the learning models whose probability value is greater than or equal to the predetermined threshold.

7. The recommended action notification system according to claim 5,
wherein the processing portion displays a recommended action display screen on a display portion of the first automatic analyzer; and
wherein the recommended action display screen displays a probability value to recommend performing the predetermined action according to the learning model as well as the name of the predetermined action recommended for an operator to perform, date and time when the predetermined abnormality is estimated to occur on the automatic analyzers, and date and time when the predetermined action is estimated to be performed on the automatic analyzers, based on an inference result concerning an average remaining time until the predetermined abnormality occurs on the automatic analyzers and an average remaining time until the predetermined action is performed on the automatic analyzers.

8. The recommended action notification system according to claim 1,
wherein the update portion groups the automatic analyzers based on the similarity of operational situations including operations and inspection contents and updates the learning model based on the learning dataset from automatic analyzers grouped based on the similarity of operational situations.

9. An automatic analyzer to perform sample analysis and maintenance, comprising:
a storage portion to store a learning model including an input layer and an output layer, wherein the input layer is supplied with related device data configured according to a predetermined action performed by an operator on the automatic analyzer and the output layer outputs an inference result related to the predetermined action in response to input of the related device data to the input layer;
a processing portion that calls the learning model from the storage portion, supplies the learning model with related device data including result data concerning one of the sample analysis and the maintenance, and recommends an operator to perform the predetermined action when a probability value is greater than or equal to a predetermined threshold, wherein the probability value recommends performing the predetermined action output from the learning model; and
a display portion that displays the name of the predetermined action recommended by the processing portion.

10. The automatic analyzer according to claim 9, comprising:
an abnormality detection portion that detects abnormalities; and
a dataset generation portion that displays an action evaluation input screen on the display portion when an operator performs the predetermined action in response to an abnormality occurrence detection from the abnormality detection portion, collects related device data corresponding to the predetermined action during a predetermined period based on the date and time to perform the predetermined action, and generates a learning dataset including the collected related device data and an effectiveness evaluation on the predetermined action and an abnormality cause input from the action evaluation input screen.

11. The automatic analyzer according to claim 10,
wherein the learning dataset is sent to a learning device and is used to update the learning model.

12. A recommended action notification method for a recommended action notification system including a plurality of automatic analyzers including a first automatic analyzer and a learning device networked to the automatic analyzers, the method allowing the recommended action notification system to recommend an operator performing an action on the first automatic analyzer,
wherein the automatic analyzers transmit a learning dataset to the learning device;
wherein the learning device updates a learning model based on the learning dataset from the automatic analyzers;
wherein the learning device delivers the updated learning model to the first automatic analyzer;
wherein the first automatic analyzer performs one of sample analysis and maintenance; and
wherein the first automatic analyzer supplies the learning model with related device data including result data concerning one of the sample analysis and the maintenance and recommends an operator to perform the predetermined action when a probability value is greater than or equal to a predetermined threshold, while the probability value recommends performing a predetermined action the learning model outputs in response to input of the related device data.

13. The recommended action notification method according to claim 12,
wherein, when one of the automatic analyzers detects an abnormality occurrence, the automatic analyzer detecting the abnormality occurrence notifies an operator of the detection of the abnormality;
wherein, when an operator performs the predetermined action in response to a notification of the abnormality, the automatic analyzer detecting the abnormality occurrence displays an action evaluation input screen on a display portion of the automatic analyzer; and
wherein the automatic analyzer detecting the abnormality occurrence collects related device data corresponding to the predetermined action during a predetermined period based on the date and time to perform the predetermined action and generates a learning dataset including the collected related device data and an effectiveness evaluation on the predetermined action and an abnormality cause input from the action evaluation input screen.

14. The recommended action notification method according to claim 12,
wherein the learning device groups the automatic analyzers based on the similarity of operational situations including operations and inspection contents and updates the learning model based on a learning dataset from automatic analyzers grouped based on the similarity of operational situations.
